# DEMANDE DE BREVET EUROPEEN

(11) **EP 0 662 517 A1**
(43) Date de publication de la demande: **12.07.1995**
(21) Numéro de dépôt: 94420373.6
(22) Date de dépôt: 30.12.1994
(51) Int. Cl.: C12Q 1/16, C12M 1/34

(54) **Procédé d'identification de micro-organismes, ses applications à la détection précoce de pousse bactérienne, et à l'optimisation de la composition des milieux de culture, et dispositif pour la mise en oeuvre de ce procédé**

(30) Priorité: 05.01.1994 FR 9400180
(71) Demandeur: INBIOMED INTERNATIONAL, F-69008 Lyon (FR)
(72) Inventeur: Guilluy, Roger, F-69720 Saint Bonnet de Mure (FR); Brazier, Jean-Louis, F-69500 Bron (FR)
(74) Mandataire: Monnier, Guy

(57) **Abrégé**

Selon ce procédé, on effectue, par spectrométrie de masse isotopique (IRMS), la mesure des fractionnements isotopiques induits sur le ¹³CO₂ dégagé à partir d'une culture sur un milieu de composition isotopique standardisée.

Applications dans le diagnostic précoce de pousse bactérienne et dans l'étude et l'optimisation des milieux de culture, .

Le dispositif pour la mise en oeuvre du procédé comporte un système compact réunissant une étuve 2 comportant des récipients d'ensemencement 3, des dispositifs d'agitation et un système de transfert des échantillons gazeux 4, ledit système étant associé à un dispositif 5 d'analyse isotopique par spectrométrie de masse isotopique, couplé à un chromatographe en phase gazeuse 6, une console informatique 8 gérant les séquences et résultats d'analyse.

## Description

La présente invention concerne un procédé d'identification de micro-organismes basé sur la mesure du ¹³CO₂ dégagé par une culture biologique, ses applications à la détection précoce de pousse bactérienne, et à l'optimisation de la composition des milieux de culture, ainsi qu'un dispositif pour la mise en oeuvre du procédé.

On sait qu'un organisme vivant qui consomme des substrats nutritifs les transforme, en fin de chaîne métabolique, en molécules simples et notamment en CO₂, ce CO₂ étant ensuite éliminé dans l'air environnant.

Différentes méthodes ont déjà été proposées pour mesurer ce dégagement de CO₂ dans des cultures biologiques et l'appliquer à des analyses microbiologiques.

C'est ainsi que Neil M. Dixon et Douglas B. Kell in Jal of Microbiological Methods, 10 (1989) pp. 155-176 passent en revue les principales méthodes utilisées pour mesurer le dégagement de CO₂ durant les processus de fermentation: méthodes titrimétrique, colorimétrique, gravimétrique, volumétrique, manométrique, mesures des différences de conductivité thermique, mesures en spectroscopie de masse IR, chromatographie, potentiométrie, conductométrie, ampérométrie, fibres optiques, mesures piézoélectriques.

D'autres méthodes utilisent l'isotope radioactif ₁₄C. On peut citer J.R. Waters (USP 3.935.073) qui propose une méthode pour traiter les bactéries, K.Brooks et Th. Sodeman in A.J.C.P. vol. 61, Juin 1974 pp 859-866 qui l'utilisent pour la détection rapide de bactérémie, F. Deland et H. N. Wagner Jr in J. Lab. Clin. Med - Mars 1970 - pp. 529-534 qui déterminent la croissance bactérienne dans les cultures de sang et H. Bopp et P.D. Ellner in Jal of Clinical Microbiology - May 1988 pp. 919-922 qui s'en servent pour évaluer des substrats pour la détection radiométrique des bactéries dans les cultures de sang.

Toutes ces méthodes présentent de nombreux inconvénients et la mesure absolue de la variation de concentration en CO₂ dans une atmosphère ne présente pas un caractère de sensibilité suffisante pour l'évaluation de nombreux processus microbiologiques.

On sait que, comme toutes les molécules organiques carbonées, le substrat nutritif consommé par les organismes vivants possède une composition isotopique en carbone (CIC), exprimée par son rapport ₁₃C/₁₂C (1,08 % du carbone ₁₃C en moyenne).

Les molécules de CO₂ issues de ce substrat, après consommation par un organisme vivant, présenteront donc un rapport ₁₃C/₁₂C qui sera le reflet des caractéristiques isotopiques dudit substrat.

En particulier, si l'un des composants du milieu de culture est volontairement enrichi en carbone 13, le métabolisme de ce substrat par un microorganisme se traduira par la libération de ₁₃ CO₂, et donc par une modification significative du rapport ₁₃ CO₂/₁₂ CO₂

US-A-4 182 656 décrit un procédé permettant de détecter la présence d'un micro-organisme. Selon ce procédé, on utilise un milieu de culture "X" dont l'un seul des composants a été enrichi avec du ¹³C. La métabolisation de ce substrat par un micro-organisme libère des quantités significatives de ¹³CO₂ qui modifient le rapport ¹³C / ¹²C ce qui peut être facilement mesuré avec les différentes méthodes classiques proposées (spectromètre de masse, spectroscopie infrarouge, laser heterodynre, spectrolaser..).

De même, EP-A-0253927 décrit un procédé de mesure de la variation du rapport ₁₃C0₂/₁₂C0₂ qui ne vise qu'à détecter la présence de microorganismes produisant l'uréase.

Par ailleurs, deux organismes vivants différents (espèces, sous-espèces...) qui utilisent pour leurs besoins nutritifs le même substrat carboné, dont la composition isotopique est connue, pourront le transformer en molécules de CO₂ de compositions isotopiques carbonées différentes, du fait de processus ou de mécanismes différents qui induisent un fractionnement isotopique.

On peut traduire ceci par le schéma suivant : 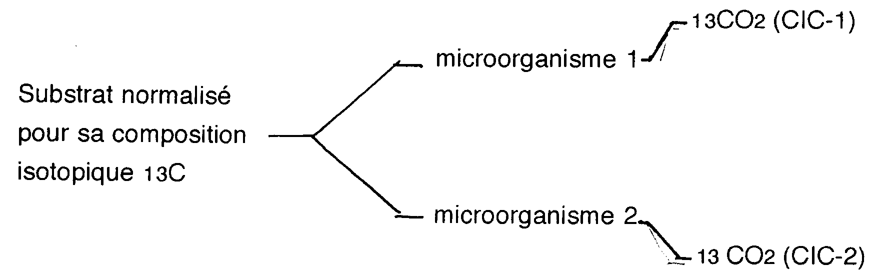
Or, les variations d'enrichissement induites par le fractionnement isotopique sont très faibles (variations au niveau de la sixième ou la cinquième décimale du rapport ₁₃C/₁₂C). Ces variations ne peuvent être acessibles par les méthodes classiques, telles, par exemple, que celles décrites dans US-A-4 182 656.

Les inventeurs ont découvert que la mesure de ce rapport avec un très haut niveau de précision, associée à une procédure normalisée, permettait de quantifier le fractionnement isotopique induit par les espèces bactériennes.

Ce fractionnement isotopique est spécifique de chaque espèce bactérienne et permet son identification.

La quantification du fractionnement isotopique est obtenue par la mesure du rapport ₁₃C/₁₂C qui consiste à compter dans un échantillon de molécules pures le nombre des atomes de l'isotope 13 et le nombre des atomes de l'isotope 12 du carbone.

Seule l'analyse isotopique par spectrométrie de masse isotopique ou IRMS (Isotope Ratio Mass Spectrometry) permet de faire ce dénombrement à un très haut niveau de précision. Cette technique consiste à mesurer l'intensité des faisceaux d'ions correspondant aux isotopomères de CO₂ de masse: 44-45 et 46 à l'aide d'un spectromètre de masse à champ fixe et collecteurs multiples (un par faisceau d'ions). Cette mesure d'intensité des faisceaux d'ions permet de calculer le rapport ₁₃C/₁₂C (précision ± 0,0002 At % ₁₃C).

L'invention concerne donc un procédé d'identification de micro-organismes basé sur la mesure du ¹³CO₂ dégagé par une culture biologique, caractérisé en ce que l'on effectue, par spectrométrie de masse isotopique (IRMS), la mesure des fractionnements isotopiques induits sur le ¹³CO₂ dégagé à partir d'une culture sur un milieu de composition isotopique standardisée.

Selon l'invention, en effet, on utilise un milieu de culture dont chacun des composants a été contrôlé et mesuré dans sa composition isotopique, de manière à disposer d'un milieu standardisé dans cette composition isotopique.

A partir de cette composition standard, les micro-organismes vont induire, par leur spécificité métabolique une différence entre le ¹³C et le ¹²C. Cette différence est propre à chaque espèce et va permettre de les identifier. Ces différences sont très faibles et ne sauraient être décelées par les méthodes classiques. La seule technique permettant de les mesurer est comme dit plus haut, la speçtrométrie de masse isotopique (IRMS). C'est grâce à cette technique IRMS que les inventeurs ont pu mettre en évidence des différences minimes, c'est-à-dire le fractionnement isotopique et c'est sur cette notion de fractionnement isotopique que repose l'originalité de l'invention.

L'application prioritaire de l'invention est de pouvoir réaliser l'identification d'espèce sur des micro-organismes.

La détection précoce pour la mise en évidence de la présence de micro-organismes est une conséquence logique de ce qui précède : en effet, la méthode de mesure est au minimum 20 à 50 fois plus sensible que les méthodes classiques.

Dans la démarche diagnostic il est en effet important de pouvoir associer détection précoce et identification des germes pathogènes. Seule l'invention peut apporter, vis-à-vis de ces deux options des réponses quasi simultanées.

Le procédé d'identification des souches bactériennes selon l'invention comporte les étapes suivantes, effectuées dans un récipient fermé surmonté d'une atmosphère gazeuse :
- Utilisation d'un milieu de culture de composition isotopique standardisée
- Mesure par IRMS de la composition isotopique carbonée (CIC) du CO₂ de l'atmosphère se trouvant au dessus du milieu nutritif avant ensemencement
- Ensemencement de ce milieu avec une souche bactérienne
- Mesure par IRMS du fractionnement isotopique ¹³C / ¹²C sur le CO₂ dégagé après consommation par ladite souche, ceci en référence à un milieu nutritif de composition isotopique connue.

L'ensemencement est avantageusement effectué avec une souche bactérienne inconnue dans un milieu nutritif contenant des substrats spécifiques de composition isotopique carbonée standardisé.

Les étapes du procédé selon l'invention sont alors les suivantes :
- Utilisation d'un milieu de culture de composition isotopique standardisée
- Introduction au dessus du milieu de culture d'un mélange gazeux standardisé dans sa composition et détermination, par IRMS, de l'enrichissement isotopique ¹³C de l'échantillon gazeux contenant du CO₂ se trouvant au dessus du milieu nutritif
- Ensemencement du milieu nutritif avec une souche bactérienne inconnue
- Mesure, par IRMS, en fonction du temps, de l'enrichissement isotopique ¹³C des échantillons gazeux contenant du CO₂ se trouvant au dessus du milieu nutritif.

Selon un autre mode de réalisation de l'invention, le procédé consiste à mesurer, par IRMS, la composition isotopique carbonée du CO₂ de l'atmosphère avant ensemencement, à effectuer un ensemencement (t=0), puis à mesurer, par IRMS, la composition isotopique carbonée du CO₂ à différents temps après l'ensemencement.

Le procédé s'applique alors au diagnostic précoce de pousse bactérienne.

Enfin, selon un autre mode de réalisation le procédé selon l'invention. consiste à faire varier la composition isotopique carbonée de l'un des substrats contenu dans un mélange nutritif, à ensemencer ce milieu nutritif avec un micro-organisme donné puis à corréler la composition isotopique carbonée du CO₂ dégagé à celle dudit substrat.

Le procédé trouve alors des applications dans l'étude et l'optimisation de la composition des milieux de culture.

Le procédé selon l'invention est de préférence réalisé dans un système compact réunissant un module d'analyse automatique et autonome fonctionnant par spectrométrie de masse isotopique, une étuve comportant les récipients où est réalisé l'ensemencement, associée à des dispositifs d'agitation, une console informatique gérant les séquences et résultats d'analyse.

Selon l'invention, ce dispositif d'analyse isotopique par spectrométrie de masse isotopique ou IRMS (Isotope Ratio Mass Spectrometry) permet de mesurer les valeurs du rapport ₁₃C/₁₂C du CO₂ produit par les microorganismes utilisés.

Les différentes étapes du procédé selon l'invention, dans son application à l'identification des souches bactériennes, vont maintenant être décrites.

La première étape consiste à disposer d'un milieu de culture dont la composition isotopique carbonée est contrôlée. Ceci est obtenu par la détermination de la composition isotopique des différents constituants entrant dans la préparation du milieu et par la même détermination effectuée sur le milieu de culture au terme de sa préparation.

Pour ces déterminations, les mesures isotopiques sont effectuées par les méthodes classiques d'analyse isotopique, c'est-à-dire par combustion et analyse du CO₂ purifié à partir du mélange gazeux résultant de la combustion.

L'étape suivante consiste à introduire au dessus du milieu de culture un mélange gazeux standardisé dans sa composition et à déterminer l'enrichissement isotopique ₁₃C de l'échantillon gazeux contenant du CO₂ se trouvant au dessus du milieu nutritif ; on effectue en une troisième étape l'ensemencement du milieu nutritif, la quatrième étape consistant à mesurer, en fonction du temps, l'enrichissement isotopique ₁₃C de l'échantillon gazeux contenant du CO₂.

L'utilisation cible du procédé se trouve dans l'identification des souches bactériennes.

En effet, si l'on ensemence un micro-organisme inconnu dans un milieu nutritif contenant des substrats spécifiques de CIC connue, il est possible, en mesurant la CIC du CO₂ produit par ce micro-organisme et en utilisant le principe précédent, d'en définir la nature (espèce, genre...).

L'identification se fait donc sur la base de la détermination de la CIC du CO₂ qu'il produit à partir d'un milieu nutritif de composition définie et contrôlée.

Les résultats obtenus par ensemencement de 7 souches bactériennes différentes sur un milieu isotopiquement standardisé sont rassemblés dans le tableau suivant. Ces résultats établissent la corrélation entre les valeurs d'enrichissement isotopiques obtenues au terme de la croissance et l'identification des différentes souches bactériennes.
SOUCHE 1 - 17,397 ± 0,085
SOUCHE 2 - 16,771 ± 0,128
SOUCHE 3 - 18,758 ± 0,184
SOUCHE 4 - 19,018 ± 0,040
SOUCHE 5 - 25,436 ± 0,330
SOUCHE 6 - 17,774 ± 0,232
SOUCHE 7 - 26,105 ± 0,060
FLACON TEMOIN - 35,992 ± 0,171
On constate que les mesures d'enrichissement en ₁₃CO₂ de l'atmosphère des milieux de culture de ces 7 souches par couplage chromatographie en phase gazeuse -IRMS ont donné des valeurs différentes entre les 7 souches. Ces valeurs sont toutes différentes de celle de l'enrichissement du flacon témoin.

La comparaison des enrichissements pour les souches prises deux par deux (test de student) a montré que toutes ces souches étaient significativement différentes quant à l'enrichissement en ₁₃C de leur production de CO₂.

On comprend donc bien qu'il soit possible, une fois établie une banque de données de différentes souches microbiennes, d'identifier facilement une souche inconnue par le procédé selon l'invention.

Par ailleurs, le procédé selon l'invention peut être appliqué au diagnostic précoce de pousse bactérienne.

Le couplage chromatographie en phase gazeuse-analyse isotopique par spectrométrie de masse isotopique en flux continu permet de mesurer une variation du rapport ₁₃C/₁₂C au niveau de la 6ème décimale et à partir de 10 nanomoles de CO₂. Il est donc possible d'évaluer très tôt une variation infime de ce rapport dans l'atmosphère d'une culture de micro-organismes (hémoculture...)
La procédure est la suivante :
On mesure la CIC du CO₂ de l'atmosphère avant ensemencement.

On effectue l'ensemencement (t = 0).

On mesure ensuite la CIC du CO₂ après l'ensemencement, à différents temps.

La variation de la CIC du CO₂ (ou son enrichissement provenant de la consommation d'un substrat donné) peut ainsi être détectée de façon précoce et donc signaler, de façon précoce, la posivité de la pousse du microorganisme.

Une autre application consiste à faire varier la CIC de l'un des substrats 1 contenu dans un mélange nutritif, puis à ensemencer ce milieu nutritif avec un microorganisme donné. Il se forme du CO₂ dont la CIC est corrélée à celle du substrat 1. On peut alors attribuer une spécificité de ce substrat pour le microorganisme en question.

Les résultats obtenus permettent d'optimiser, pour une meilleure spécificité, la composition des milieux nutritifs. Les données cinétiques de l'expérimentation à partir d'une souche définie permettent de relier les paramètres caractéristiques de la pousse bactérienne à la spécificité des éléments marqués introduits dans les milieux de culture, ce qui permet l'optimisation de ces milieux.

Le dessin annexé, donné à titre d'exemple, permettra de mieux comprendre l'invention, les caractéristiques qu'elle présente et les avantages qu'elle est susceptible de procurer, notamment dans le diagnostic précoce de pousse bactérienne.

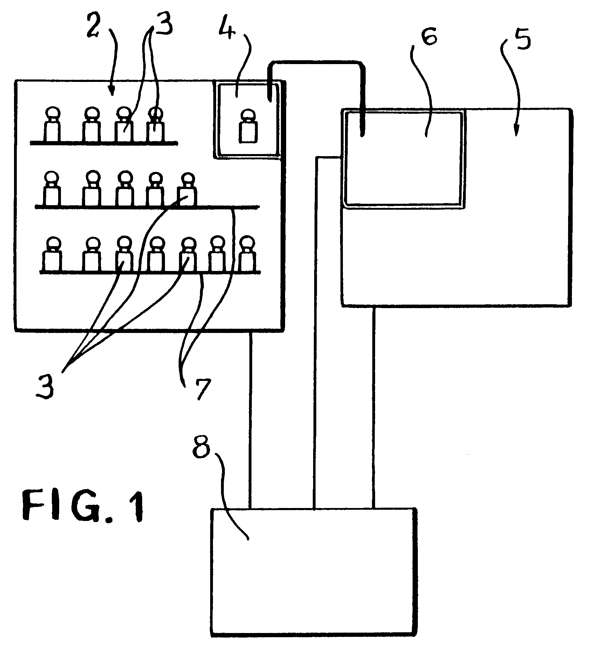

La figure 1 est une vue schématique de l'installation mise en oeuvre pour la réalisation du procédé selon l'invention.

Sur la figure 1, l'étuve est désignée par 2, et le dispositif d'analyse isotopique par IRMS par 5.

Les figures 2 à 5 représentent les profils isotopiques ₁₃C en spectrométrie de masse isotopique des produits marqués obtenus après ensemencement respectivement avec Staphyloccocus Aureus (fig. 2), Candida Albicans (fig. 3), Escherichia Coli (fig. 4) et Escherichia Coli (sang) (fig. 5).

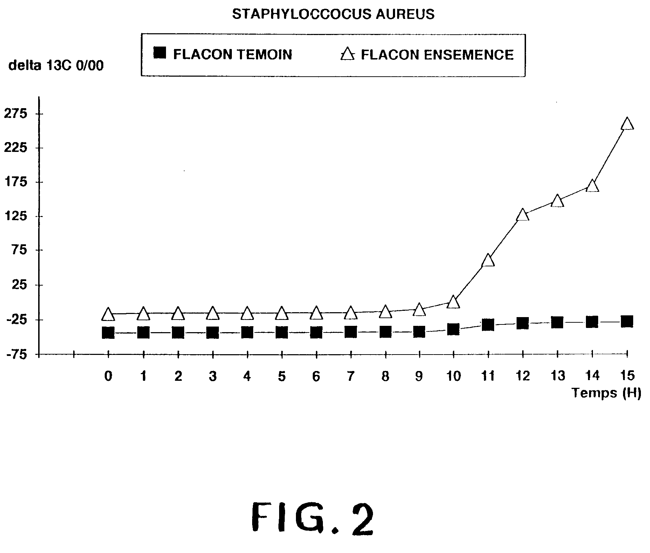

Sur les figures 2 à 5, décrivant des exemples de diagnostic précoce de pousse bactérienne, ainsi que sur les tableaux les accompagnant, le temps est exprimé en heures, et l'enrichissement isotopique en ₁₃C0₂ est donné en Delta ₁₃C 0/00 respectivement sur un milieu témoin et sur un milieu ensemencé

Le dispositif représenté à la figure 1 se compose essentiellement d'une étuve 2, équipée de plateaux 7 comportant les récipients 3 où est réalisé l'ensemencement. Des dispositifs d'agitation à mouvement orbital (non représentés au dessin ) sont prévus pour accélérer la croissance des microorganismes présents.

A cette étuve 2, fonctionnant en circuit fermé, est associé un module 4 permettant le transfert des échantillons gazeux dans un dispositif 5 d'analyse isotopique par IRMS.

C'est ce dispositif d'analyse 5 associé à un chromatographe en phase gazeuse 6 qui permet de mesurer les valeurs du rapport ₁₃C/₁₂C obtenu pour les microorganismes cultivés dans les récipients 3 disposés sur les plateaux 7 de l'étuve 2. La console informatique 8 assure la gestion des séquences et des résultats d'analyse.

**Tableau 1**

| STAPHYLOCCOCUS AUREUS Enrichissement ₁₃CO2 | | |
|---|---|---|
| Temps Heure | Flacon témoin Delta ₁₃C O/00 | Flacon ensemencé Delta ₁₃C 0/00 |
| 0 | -43,5 | -16,5 |
| 1 | -43,3 | -15,4 |
| 2 | -43,2 | -15,2 |
| 3 | -43 | -14,7 |
| 4 | -42,7 | -14,6 |
| 5 | -42,5 | -14,5 |
| 6 | -42,5 | -14,2 |
| 7 | -42,1 | -13,9 |
| 8 | -42 | -12,8 |
| 9 | -42 | - 9,5 |
| 10 | -38,9 | 1,2 |
| 11 | -32,5 | 61,3 |
| 12 | -30,4 | 128 |
| 13 | -29,5 | 149 |
| 14 | -28,8 | 171 |
| 15 | -28,4 | 263 |

La courbe de la figure 2 illustre les résultats ci-dessus.

**Tableau 2**

| CANDIDA ALBICANS Enrichissement ₁₃CO2 | | |
|---|---|---|
| Temps Heure | Flacon témoin Delta ₁₃C O/00 | Flacon ensemencé Delta 13C 0/00 |
| 0 | -43,7 | -17,2 |
| 1 | -43,6 | -16,1 |
| 2 | -43,7 | -16 |
| 3 | -43,7 | -15,1 |
| 4 | -43,4 | -13,7 |
| 5 | -43,4 | -11,6 |
| 6 | -43,4 | -11,1 |
| 7 | -42,9 | - 9,6 |
| 8 | -42,8 | - 5,7 |
| 9 | -42,6 | - 0,5 |
| 10 | -41,9 | 15,3 |
| 11 | -39,8 | 78,6 |
| 12 | -36,4 | 191,5 |
| 13 | -31,9 | 288,3 |
| 14 | -28,2 | 353,1 |
| 30 | -14,9 | 548,9 |

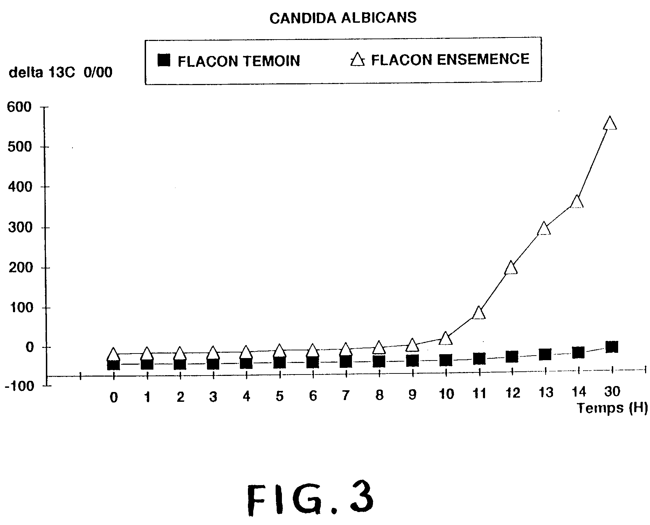

La courbe de la figure 3 illustre les résultats ci-dessus.

**Tableau 3**

| ESCHERICHIA COLl Enrichissement ₁₃CO2 | | |
|---|---|---|
| Temps Heure | Flacon témoin Delta ₁₃C O/00 | Flacon ensemencé Delta ₁₃C 0/00 |
| 0 | -44,5 | -18,2 |
| 1 | -45 | -17,6 |
| 2 | -44,7 | -16,9 |
| 3 | -44,1 | -16,7 |
| 4 | -43,9 | -16,9 |
| 5 | -43,6 | -16,4 |
| 6 | -43,3 | -15 |
| 7 | -42,9 | -12,3 |
| 8 | -41,7 | 5,5 |
| 9 | -35,3 | 17,1 |
| 10 | -26,1 | 211 |
| 11 | -21,8 | 303 |
| 12 | -19,5 | 303,1 |
| 13 | -18,3 | 389,70 |

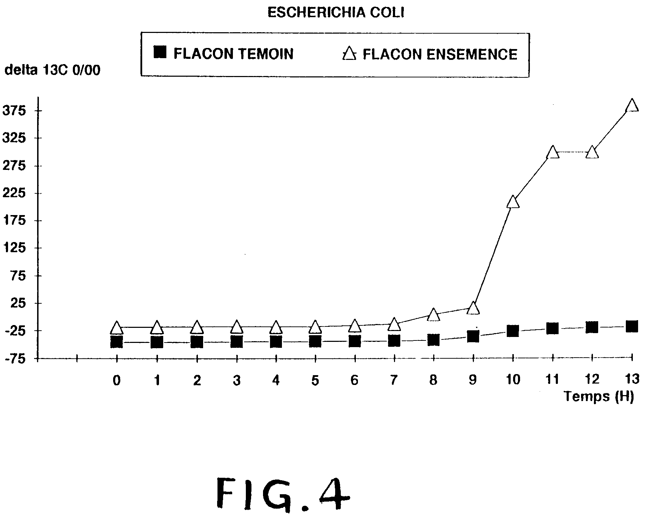

La courbe de la figure 4 illustre les résultats ci-dessus.

**Tableau 4**

| ESCHERICHIA COLl (Sang) Enrichissement 13CO2 | | |
|---|---|---|
| Temps Heure | Flacon témoin Delta 13C O/00 | Flacon ensemencé Delta 13C 0/00 |
| 0 | -44,6 | -20,9 |
| 1 | -40,9 | -21,7 |
| 3 | -40,1 | -20,2 |
| 4 | -39,6 | -19,8 |
| 5 | -39,2 | -18,2 |
| 6 | -39,1 | -17,3 |
| 7 | -37,9 | -15 |
| 8 | -37,9 | -14 |
| 9 | -37,6 | -12,4 |
| 10 | -37,2 | -11 |
| 11 | -37,2 | -10,2 |
| 12 | -37,2 | - 9,4 |
| 13 | -37,1 | - 7,5 |
| 14 | -36,8 | - 5,3 |
| 15 | -36,7 | - 2,3 |
| 27 | -18,5 | 471 |
| 32 | -18,3 | 472 |

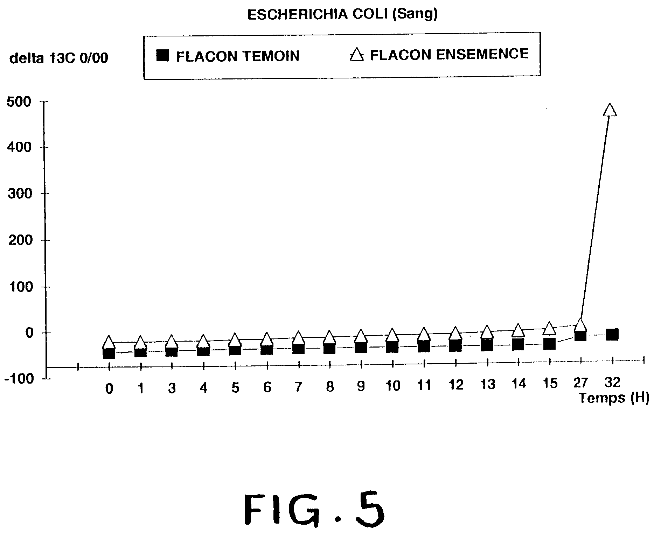

La courbe de la figure 5 illustre les résultats ci-dessus.

Des courbes similaires, réalisées dans des conditions standards et avec un milieu standard dont on connait l'enrichissement isotopique, sur des souches inconnues, permettent de corréler les valeurs d'enrichissement isotopique obtenues au terme de la croissance à la nature des différentes souches bactériennes.

De même, les données cinétiques de l'expérimentation à partir d'une souche définie permettent de relier les paramètres caractéristiques de la pousse bactérienne à la spécificité des éléments marqués introduits dans les milieux de culture, ce qui permet l'optimisation de ces milieux.

## Revendications

1. Procédé d'identification de micro-organismes caractérisé en ce que l'on effectue, par spectrométrie de masse isotopique (IRMS), la mesure des fractionnements isotopiques induits sur le ¹³CO₂ dégagé à partir d'une culture sur un milieu de composition isotopique standardisée.

2. Procédé d'identification des souches bactériennes selon la revendication 1, caractérisé en ce qu'il comporte les étapes suivantes, effectuées dans un récipient fermé surmonté d'une atmosphère gazeuse :
- Utilisation d'un milieu de culture de composition isotopique standardisée
- Mesure par IRMS de la composition isotopique carbonée (CIC) du CO₂ de l'atmosphère se trouvant au dessus du milieu nutritif avant ensemencement
- Ensemencement de ce milieu avec une souche bactérienne
- Mesure par IRMS du fractionnement isotopique ¹³C / ¹²C sur le CO₂ dégagé après consommation par ladite souche, ceci en référence à un milieu nutritif de composition isotopique connue.

3. Procédé selon la revendication 1 et la revendication 2, caractérisé en ce que l'ensemencement est effectué avec une souche bactérienne inconnue dans un milieu nutritif contenant des substrats spécifiques de composition isotopique carbonée standardisé.

4. Procédé selon l'une des revendications 1 à 3 caractérisé en ce qu'il comporte les étapes suivantes :
- Utilisation d'un milieu de culture de composition isotopique standardisée
- Introduction au dessus du milieu de culture d'un mélange gazeux standardisé dans sa composition et détermination, par IRMS, de l'enrichissement isotopique ¹³C de l'échantillon gazeux contenant du CO₂ se trouvant au dessus du milieu nutritif
- Ensemencement du milieu nutritif avec une souche bactérienne inconnue
- Mesure, par IRMS, en fonction du temps, de l'enrichissement isotopique ¹³C des échantillons gazeux contenant du CO₂ se trouvant au dessus du milieu nutritif.

5. Procédé selon la revendication 1, caractérisé en ce qu'il consiste à mesurer, par IRMS, la composition isotopique carbonée du CO₂ de l'atmosphère avant ensemencement, à effectuer un ensemencement (t=0), puis à mesurer, par IRMS, la composition isotopique carbonée du CO₂ à différents temps après l'ensemencement.

6. Application du procédé selon la revendication 5 au diagnostic précoce de pousse bactérienne.

7. Procédé selon la revendication 1 caractérisé en ce qu'il consiste à faire varier la composition isotopique carbonée de l'un des substrats contenu dans un mélange nutritif, à ensemencer ce milieu nutritif avec un micro-organisme donné puis à corréler la composition isotopique carbonée du CO₂ dégagé à celle dudit substrat.

8. Application du procédé selon la revendication 7 à l'étude et à l'optimisation de la composition des milieux de culture.

9. Dispositif pour la mise en oeuvre du procédé selon l'une quelconque des revendications 1 à 8, caractérisé en ce qu'il comporte un système compact réunissant une étuve 2 comportant des récipients d'ensemencement 3, des dispositifs d'agitation et un système de transfert des échantillons gazeux 4, ledit système étant associé à un dispositif 5 d'analyse isotopique par spectrométrie de masse isotopique, couplé à un chromatographe en phase gazeuse 6, une console informatique 8 gérant les séquences et résultats d'analyse.
